# EUROPEAN PATENT SPECIFICATION

(11) **EP 2 614 372 B1**
(45) Date of publication and mention of the grant of the patent: **25.07.2018**
(21) Application number: 11823155.4
(22) Date of filing: 07.09.2011
(51) Int. Cl.: G01N 33/545

(54) **KIT FOR MONITORING, DETECTING AND STAGING GVHD**
KIT ZUR ÜBERWACHUNG, ERKENNUNG UND EINSTUFUNG EINER TRANSPLANTAT-GEGEN-WIRT-REAKTION
KIT DE SURVEILLANCE, DÉPISTAGE ET STADIFICATION DE LA MALADIE DU GREFFON CONTRE L'HÔTE

(30) Priority: 18.01.2011 US 201161433540 P; 07.09.2010 US 380337 P
(43) Date of publication of application: 17.07.2013
(73) Proprietor: Marx, Stephen G., 97761 Jerusalem (IL)
(72) Inventor: Marx, Stephen G., 97761 Jerusalem (IL)
(74) Representative: Lecomte & Partners
(86) International application number: PCT/IL2011/000711
(87) International publication number: WO 2012/032511

(56) References cited:
- EP-A1- 2 163 896
- EP-A2- 1 491 894
- WO-A1-2006/006939
- US-A1- 2003 228 618
- US-A1- 2004 101 907
- US-A1- 2005 250 178
- US-A1- 2009 104 628
- KLAMER G D H ET AL: "INVESTIGATING THE POTENTIAL OF PHARMACOCHEMICAL INHIBITION OF GSK3-BETA TO REDUCE THE BURDEN OF GVHD AFTER ALLOGENEIC HEMATOPOIETIC STEM CELL TRANSPLANTATION", EXPERIMENTAL HEMATOLOGY (NEW YORK), vol. 38, no. 9, Suppl. 1, 5 September 2010 (2010-09-05), page S35, XP002722108, & 39TH ANNUAL SCIENTIFIC MEETING OF THE ISEH - SOCIETY-FOR-HEMATOLOGY-AND-STEM-CELLS; MELBOURNE, AUSTRALIA; SEPTEMBER 15 -18, 2010 ISSN: 0301-472X
- ORBACH A ET AL: "Utilizing glycogen synthase kinase-3[beta] as a marker for the diagnosis of graft-versus-host disease.", TRANSPLANTATION PROCEEDINGS JUN 2013, vol. 45, no. 5, June 2013 (2013-06), pages 2051-2055, XP002722109, ISSN: 1873-2623

## Description

### BACKGROUND OF THE INVENTION

### 1. Field of the Invention

The present invention is in the field of diagnostic and regulatory kits for the treatment of conditions associated with pathological cell proliferation. More specifically, the invention is adapted to measure and regulate the activation and inactivation levels of glycogen synthase kinase 3 (GSK-3) as a marker of T-cell proliferation, especially in conditions such as Graft versus Host Disease (GVHD).

### 2. Background Art

Glycogen synthase kinase 3 (GSK-3) is a serine/threonine kinase that is known to act as a downstream regulatory switch that determines the output of numerous signaling pathways initiated by diverse stimuli. Through these pathways GSK-3 plays a central role in cellular proliferation and apoptosis. The ability to detect the activation state of the GSK-3 and if necessary to regulate it, would allow a greater understanding and ability to treat and diagnose conditions related to cell proliferation and apoptosis.

In many conditions related to the immune response, including tissue rejection, inflammation, infection and GVHD, there is a marked change in cell proliferation, in particular an increase in T-cell proliferation.

The incidence of GVHD has increased substantially over recent years. The number of allogeneic hematopoietic stem cell transplants (HSCT) that occur annually continues to increase each year. GVHD may also occur occasionally as a complication of blood transfusion. Clinically, the diagnosis of GVHD is suspected when the patient develops one or more of the following observable symptoms: dermatitis (skin rash), cutaneous blisters, crampy abdominal pain with or without diarrhea, persistent nausea and vomiting, hepatitis (with elevation of bilirubin and/or liver enzymes) (Jacobsohn D A and Vogelsang G B 2007). Clinical observations along with current diagnostic techniques are time-consuming and require invasive tissue sampling, involving tissue biopsies obtained from an involved organ in order to confirm and diagnose GVHD. Although biopsies are the only way to properly diagnose GVDH today, the diagnosis is determined only after T cells are already attacking the host tissues and the GVHD process has reached phase 3 of the disorder. Biopsy procedures to procure tissue are also invasive and incur delays of 24 to 48 hours before a diagnosis can be rendered. Furthermore, an unequivocal diagnosis is not always possible with biopsies, and diagnosis from clinical symptoms is not reliable, given that other post-transplant conditions may present in a similar manner. Certain biomakrers for GVHD have been described in the prior art. For instance EP 2 163 896 shows that CCL8 is a marker for the early diagnosis of GVHD.

There is therefore a long-felt need to provide a rapid, reliable relatively non-invasive method for detecting GVHD and other pathological conditions involving cell proliferation in the early stages of the disease process.

### SUMMARY OF THE INVENTION

The present invention relates to the field of diagnostic and regulatory kits and more specifically to a diagnostic and regulatory kit for Graft versus Host Disease.

The present invention relates to a method of detecting GVHD in a mammal including a human in accordance with appended independent claim 1 and appended dependent claims 2-8.

The present invention also relates to an use of a kit in the diagnosis of GVHD in accordance with appended independent claim 9 and appended dependent claim 10.

### BRIEF DESCRIPTION OF THE DRAWINGS

The invention is herein described, by way of example only, with reference to the accompanying drawings. The particulars shown are by way of example only and for the purposes of illustrative discussion of the preferred embodiments of the present invention only. The description taken with the drawings should make apparent to those skilled in the art how several forms of the invention may be embodied in practice.

In the drawings:
Figure 1 is a pictorial illustration of the GSK3α and GSK3β subunits and their known activation and inactivation sites;
Figure 2 is a flow diagram connecting GSK3β to GVHD;
Figure 3 is a pictorial illustration of the disease process in GVHD;
Figure 4A is a graphical representation of ser 9- phosphorylated GSK-3β (p-GSK-3β) protein expression levels in spleen samples;
Figure 4B is a graphical representation of total GSK-3β protein expression levels in spleen samples;
Figure 5 is a graphical representation of phosphorylated PKC (p-PKC) protein expression levels in spleen samples;
Figure 6A is a graphical representation of ser 9- phosphorylated GSK-3β (p-GSK-3β) protein expression levels in blood samples; and,
Figure 6B is a graphical representation of total GSK-3β protein expression levels in blood samples.

### DETAILED DESCRIPTION OF THE INVENTION

The present invention solves many of the problems of current methods and systems for monitoring patients suffering from conditions associated with pathological cell proliferation and pathological cell apoptosis especially GVHD.

It is therefore a preferred embodiment of the present invention to disclose a diagnostic kit, capable of detecting the expression level of glycogen synthase kinase 3β (GSK-3β). In this embodiment the GSK-3β expression level is an indicator of GVHD in the patient.

GSK-3β is now known to act as a downstream regulatory switch that determines the output of numerous signaling pathways initiated by diverse stimuli (Frame and Cohen 2001, Grimes and Jope 2001, Woodgett 2001 and Doble and Woodgett 2003).

The activation status of the GSK-3β, the expression levels of the GSK-3β or the site on the GSK-3β responsible for activating or inactivating of the are used to detect and monitor GVHD.

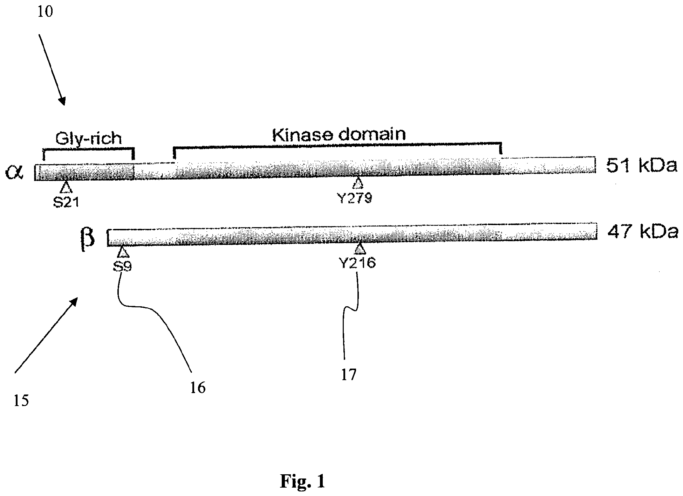

Reference is now made to Figure 1 in the drawings, which shows the two subunits of GSK-3, GSK-3α **10** and GSK-3β **15** and their phosphorylation sites. Specific reference is made to the GSK-3β subunit, in which known phosphorylation sites, including the serine 9 (S9) **16** and tyrosine 216 (Y216) **17,** are shown. In this embodiment the normal physiological state of the GSK-3β is in an unphosphorylated or dephosphorylated active state. In this embodiment serine 9 phosphorylation inactivates the GSK-3β, which can be identified and quantitated according to the methods described forthwith in the description. Serine 9 phosphorylation is associated with various other proteins including but not limited to cyclic adenosine monophosphate (cAMP), Phosphoinositide 3-kinase (PI3K), Phosphatidylinositol 4,5-biphosphate (PIP₂), protein kinase A (PKA) and Akt (serine/threonine protein kinase family).

In a further embodiment of the present invention, GSK-3β inactivation may also occur through the Wnt signaling pathway. Wnt activation inactivates the GSK-3β through an inactivation site independent of serine 9 phosphorylation and is identified and quantitated through measuring changes in non-phosphorylated β-catenin levels. In a further embodiment activation of the GSK-3β is either through dephosphorylation or through phosphorylation of tyrosine 216 (Y216). GSK-3β phosphorylation can be identified and quantitated through changes in the molecular weight of the GSK-3β using methods described forthwith in the description. In one aspect of this embodiment the diagnostic kit of the present invention uses antibodies to identify and quantify GSK-3β expression levels.

It is a preferred embodiment of the present invention that GSK-3β is a diagnostic and/or therapeutic molecule incorporated into a diagnostic and/ or therapeutic kit for detection and monitoring of GVHD.

It is a preferred embodiment of the diagnostic kit of the present invention to characterize GSK3β activity by detecting and quantifying the protein levels of total GSK3β and GSK3β phosphorylated at ser 9. Also in this embodiment, the diagnostic kit is able to detect and quantify GSK3β inactivation through activated protein kinase C (PKC) protein level and GSK3β inactivation through activated Wnt.

Thus it is a preferred embodiment of the present invention to identify GSK-3β associated molecular markers, for example proteins that are affected by the activated Wnt pathway or stimulated by activated PKC to increase the accuracy of GVHD detection. the site on the GSK-3β that is affected by the activated Wnt pathway and the site on the GSK-3β that is stimulated by activated PKC.

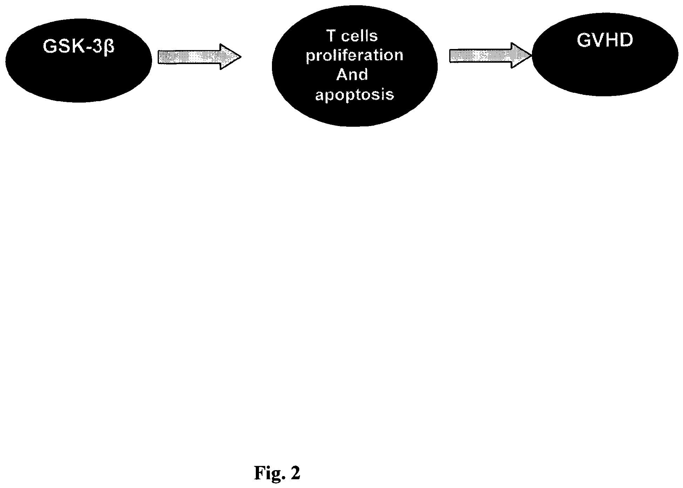

Abnormal cell proliferation and apoptosis is a main feature of Graft versus host disease (GVHD) and other conditions related to immune responses in which there is proliferation of T-cells as part of the patient's response to a foreign body or bodies as shown in Figure 2 of the drawings. In this embodiment and other preferred embodiments the diagnostic molecule and kit is used and has applications in GVHD. It is a further embodiment of the present invention that regulation of GSK-3β activation status by identification and quantification of the expression levels and inactivation and activation status ofGSK-3β and its associated pathways is adapted to regulate and monitor GVHD. In this embodiment GSK-3β expression level is detected and quantified by the diagnostic kit of the present invention.

A diagnostic kit capable of identifying GVHD in at least phase 2 of the disease by identifying and quantifying the GSK-3β expression and activation status, is a further embodiment of the present invention. In this embodiment the diagnostic kit measures a range of parameters associated with GSK-3β activation states and associated pathways, and compares the deviation from standard measurements taken from healthy subjects in order to determine the extent of GVHD progress and make the appropriate diagnosis of GVHD.

The diagnostic kit of the present invention is adapted to detect the early onset of GVHD by examining changes in the GSK-3β activity by measuring or determining the expression levels of GSK-3β and related proteins Furthermore in this embodiment the diagnostic kit and methods of the present invention will be able to determine the cause of the GVHD with regard to T cell proliferation and apoptosis by determining how the GSK-3β regulates the aforementioned proliferation and apoptosis. In this aspect of the preferred embodiment the information provided by the diagnostic kit is used for determining the most effective treatment for the GVHD through regulating activation and/or inactivation status of the GSK-3β.

It is a further preferred embodiment of the present invention that the diagnostic kit is adapted to detect protein profiles specific and uniquely characteristic to GVHD so as to be able to make a correct and accurate diagnosis of GVHD. In this embodiment the unique protein profile prevents the diagnosis of false positive GVHD.

It is therefore a preferred embodiment of the present invention that GSK-3β acts as a regulatory and therapeutic molecule in a therapeutic or pharmaceutical kit in applications including but not limited to GVHD and tissue rejection.

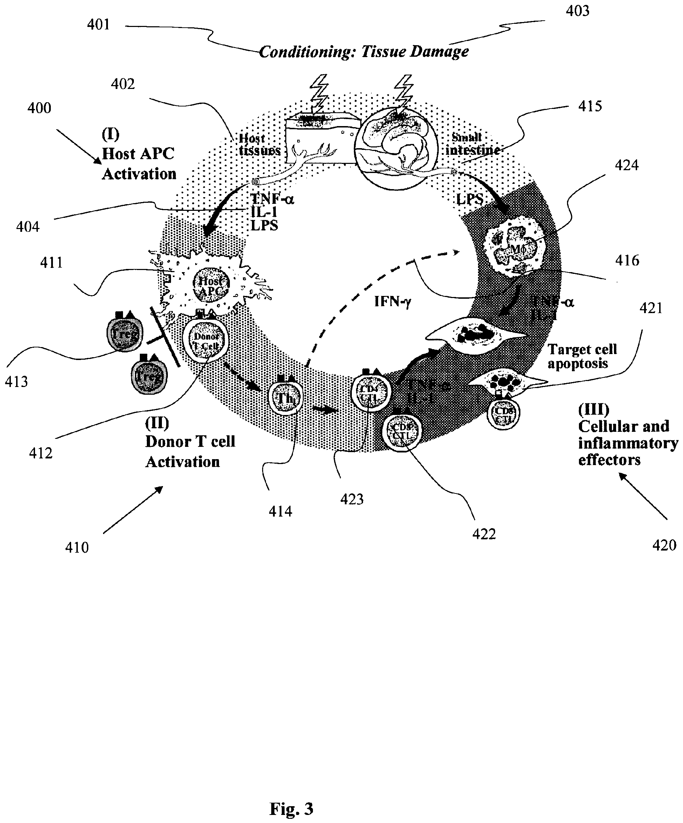

Reference is now made to Figure 3 in the drawings in which 3 phases of GVHD disease progression are shown. In phase I **400,** antigen presenting cells (APC) are activated, in the process of the recipient conditioning regimen **401,** host tissue **402** is damaged **403,** causing the release of inflammatory cytokines **404** such as Tumor Necrosis Factor α (TNF-α), Interleukin-1 (IL-1), Interleukin-6 (IL-6) and Lipopolysaccharide (LPS) immune complexes. Increased levels of these cytokines leads to activation of the host APC's. In phase II of GVHD **410** donor T-cell activation occurs, the host APC's **411** activate donor T-cells **412,** this may be prevented by regulatory T cells (Treg) **413.** The subsequent increased survival (i.e. decreased apoptosis), proliferation, migration **416** and differentiation of the activated T-cells in phase II of GVHD produces additional effectors that mediate further tissue damage including cytotoxic T Lymphocytes, Natural Killer (NK) cells, TNFα and IL-1. LPS that has leaked through the damaged intestinal mucosa **415** triggers additional TNFa production. TNFα can damage tissue directly by inducing necrosis and apoptosis in the skin and gastrointestinal tract through either TNF receptors or the Fas pathway. TNFα plays a direct role in intestinal GVHD damage which further amplifies damage in the skin, liver and lung in a "cytokine storm". In addition type 1 T helper cells (Th1) **414** are also released in phase II to facilitate the immune response by releasing cytokines and helping in presenting antigens to the T-cells. In phase III of GVHD **420,** T-cells target the host tissue for destruction with a range of cellular and inflammatory effectors. Target cells **421** undergo apoptosis and CD4 **423,** CD8 **422** and CTL expressing T-cells are present in the immune response. Macrophages **424** are mediated by LPS and interferon-γ (IFN-γ) in phase 3 of the GVHD disease process. Once activated the macrophages initiate a cascade of inflammatory responses, including the release of mediators of tissue injury - effecting in particular tissues and organs that undergo constant proliferation- such as the gastrointestinal tract and the skin.

It is an embodiment of the present invention that a diagnostic kit capable of detecting and quantitating the expression and activation status of the GSK-3β (GSK-3β activation levels and GSK-3β inactivation levels) is able to diagnose GVHD during phase II of the disease, at the stage when T-cell proliferation and migration occur. The novel diagnostic kit is able to diagnose GVHD at a relatively early stage before extensive tissue damage occurs, in a quick and relatively non-invasive manner.

In a further embodiment of the present invention, the diagnostic kit for GVHD is able to determine the status of GSK-3β as measured by examining GSK-3β expression levels. The diagnostic kit of the present invention is adapted to accurate and differential diagnosis of GVHD by further detection of unique RNA or protein profiles for GVHD, so as to prevent misdiagnosis with other conditions involving T-cell proliferation and activation.

In another embodiment of the invention, a protein chip utilizing an array of antibodies, biomarkers or antigens bound to a glass slide or equivalent substrate is used to determine the expression levels of different GVHD relevant proteins in a given sample.

In another embodiment of the invention, a diagnostic kit based on a protein chip is used to diagnose GVHD before the appearance of clinical symptoms. In certain embodiments the kit contains a custom-made protein chip comprising antibodies which are adapted to monitor the activation state and/ or expression level of the GSK-3β, related pathways, apoptosis markers, proliferation markers, and other biological and biochemical molecules. Such molecule candidates can be pre determined and analysed by western blot, FACS, and other fingerprinting techniques.

In other preferred embodiments of the invention, the establishment of the GVHD diagnostic kit and its efficacy is tested by the following stages:
- GVHD mouse model - samples from mice are utilized to determine the array of antibodies that is loaded on the protein chip. The aforementioned chip is then tested on mouse samples.
- Clinical trials - used to determine the efficacy of the protein chip (diagnostic kit prototype) and to predict the development of GVHD in human transplant patients.

In order to understand the invention and to see how it may be implemented in practice, a plurality of preferred embodiments will now be described, by way of nonlimiting example only, with reference to the following examples.

Reference is now made to experiments made in a mouse model to determine the involvement of GSK-3β and other proteins from related pathways in GVHD and their potential use as markers for GVHD diagnosis at an early disease stage.

### Goal:

To demonstrate that the levels of GSK-3β and proteins from related pathways can be used as markers for GVHD diagnosis at an early disease stage.

### GVHD experimental mouse model:

Samples were collected from blood and spleen on days 0, 1, 3, 4, 5, & 6 post-transplant, from the following 3 groups (at least 3 mice per group):
1. **Untreated:** injected with of plain medium (no irradiation). Expected result: healthy mice.
2. **Syngeneic:** Balb/c mice injected with a splenocyte transplant from matched donor Balb/c mice after irradiation. Expected result: short-term effects of irradiation and full recovery, no GVHD.
3. **Allogeneic:** Balb/c mice injected with a splenocyte transplant from unmatched donor C57B1/6 mice after irradiation. Expected result: short term effects of irradiation and subsequent GVHD development.

### Summary of Results:

- The levels of p-GSK-3β (ser-9 phosphorylated GSK-3β) and total GSK-3β are much lower in GVHD mice than in both syngeneic and untreated control mice.
- The abovementioned effect is seen over time in day's 3-6 post transplant.

### EXAMPLE 1

### GVHD mouse model: Spleen results

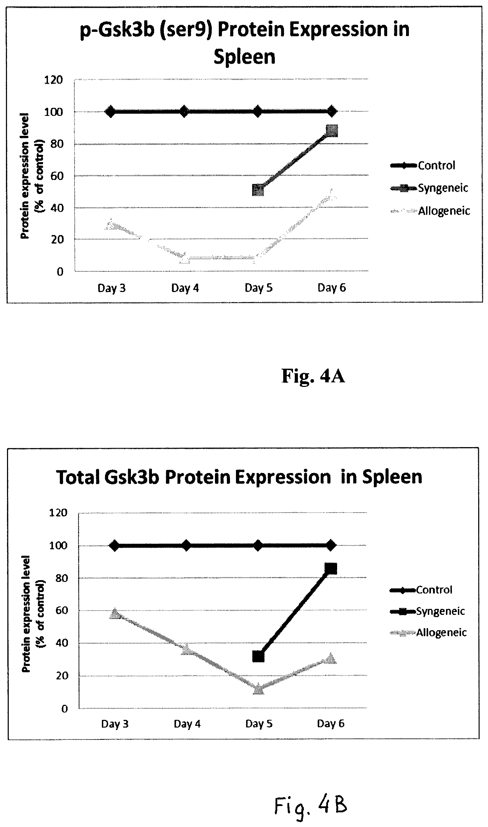

Reference is now made to **Figure 4** showing a graphical representation of the protein expression levels of p-GSK-3β (phosphotylated at ser 9 site) (Figure 4A) and total GSK-3β (Figure 4B) over time in spleen samples derived from Untreated (control), Syngeneic and Allogeneic mice.

The results have been normalized such that the values for the control mice were set as 100%. Each parameter was compared to the values of untreated mice.

As shown in **Figure 4****,** a prolonged decrease of total GSK-3β and p-GSK-3β over time was exclusively detected in the spleen of the allogeneic group mice.

It should be mentioned that in the Syngeneic group, the splenocyte population has not recovered enough to collect cells, until day 5.

In the Allogeneic group, proliferation of splenocytes was detected in the earlier days as a result of the immune response characteristic of GVHD.

On days 5 and 6, once severe symptoms have appeared in the mice, the levels of total and phosphorylated GSK-3β in the allogeneic mice were maintained at a lower level than the other groups, while in the syngeneic mice, the levels appear to approach almost normal.

Thus the results obtained show that, in the allogeneic group, p-GSK-3β and total GSK-3β decrease before the appearance of clinical symptoms (symptoms begin on day 4) and maintain lower than the untreated and Syngeneic groups throughout the disease course.

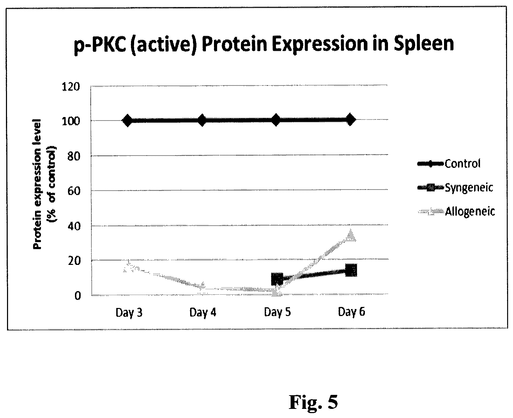

Reference is now made to **Figure 5** showing a graphical representation of the protein expression levels of p-PKC (active form), over time in spleen samples derived from Untreated (control), Syngeneic and Allogeneic mice.

It is herein acknowledged that PKC is located upstream of GSK-3β in several pathways, and is also capable of phosphorylating GSK-3β directly.

As shown in **Figure 5**, the activated PKC levels are significantly lower in splenocytes of both Syngeneic and Allogeneic mice, relative to the control.

It should be emphasised that the results obtained have been normalized such that values of the control mice were set as 100%. Each parameter was compared to the value of untreated mice independently of other parameters.

### Conclusions for the spleen results:

- The differences in the total GSK-3β and the p-GSK-3β in the spleen of the allogeneic mice compared to the syngeneic mice, can be detected as early as day 3, thus these proteins can be utilized as markers to identify and diagnose the early onset of GVHD in mice and potentially in humans.
- Based on the above results, a diagnostic kit utilizing protein chip technology can be used to monitor the changes in the GSK-3β expression levels and changes in the activation state of GSK-3β in the spleen in order to predict and detect the early onset of GVHD in humans.

### EXAMPLE 2

### GVHD mouse model: Blood results

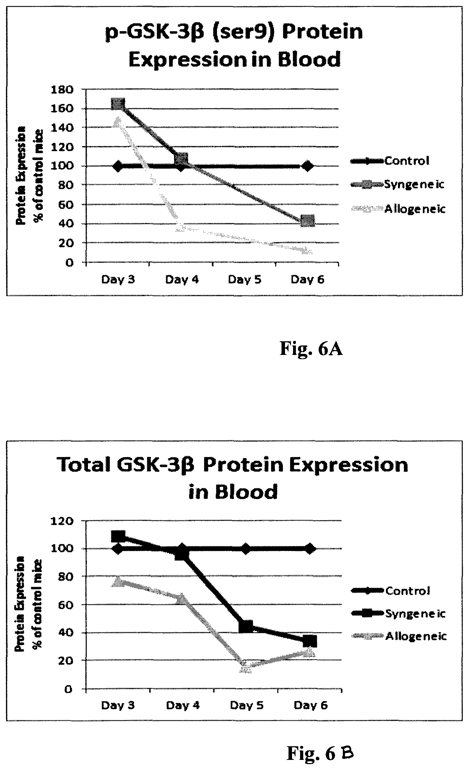

Reference is now made to **Figure 6** showing a graphical representation of the protein expression levels of p-GSK-3β (phosphotylated at ser 9 site) (**Figure 6**A) and total GSK-3β (**Figure 6**B) over time in blood samples derived from Untreated (control), Syngeneic and Allogeneic mice.

As shown in **Figure 6****,** a prolonged decrease of total GSK-3β and p-GSK-3β in the blood was observed over time only in the allogeneic group.

In the Syngeneic group, although a decrease in total GSK-3β and p-GSK-3β is observed, it appears in a later phase than in the allogeneic mice.

In the Allogeneic group, there is a decrease in the levels of total GSK-3β and p-GSK-3β over time, which is greater than the decrease observed in the syngeneic group.

It should be mentioned that the results have been normalized such that values for the control mice were set as 100%. Each parameter was compared to the values of the untreated mice.

### Conclusions for the blood results:

- The decrease in total GSK-3β and p-GSK-3β in the blood detected in the allogeneic mice was greater than both the control and the syngeneic mice.
- The decrease is most substantial on days 3 and 4, which are at the early stage of GVHD.
- Based on the above results, a diagnostic kit utilizing protein chip technology can be used to monitor the changes in the GSK-3β expression levels and changes in the activation state of GSK-3β in the blood in order to predict and detect the early onset of GVHD in other mammals including humans.
- Blood samples indicative of GVHD were obtained from mice, demonstrating the ability to collect usable blood samples from other mammals including humans.
- Without wishing to be bound by theory, it is herein acknowledged that the detected blood levels in mice reflect that GVHD associated proliferation occurred in the spleen. Thus it can be assumed that the cells observed in the syngeneic mice on days 3, 4, and maybe 5 are cells that have existed in the periphery before the transplant. This is in contrast to the cells observed in the allogenenic mice on day 4, which are new cells that have been emerged from the spleen.

The above results demonstrate for the first time that GSK-3β is directly associated with the progression and onset of the GVHD disorder.

### EXAMPLE 3

### Fingerprinting GVHD disease

A further main aspect of the present invention is to explore the unique fingerprint of the GVHD disease. Such a fingerprint allows the establishment of a diagnostic kit as disclosed *inter alia* which comprises a set of proteins that are uniquely correlated with GVHD. In order to determine the fingerprint for GVHD, mouse model samples are analyzed by mass spectrometry to identify the following data:
- The proteins that are present in the analysed system
- Changes in protein expression
- Changes in protein phosphorylation

Based on the analysis of the mouse samples, a custom-made protein chip is prepared. The kit includes candidate molecules identified to be involved in GVHD such as antibodies against GSK-3β, or proteins involved in related pathways, apoptosis markers, proliferation markers, and others (obtained by using techniques such as western blot analysis, FACS, and fingerprinting data). The aforementioned kit is used for prognosis of GVHD and its diagnosis in early stages.

Candidate antibodies used for the GVHD protein chip include antibodies recognizing the following biomarkers:
- Total GSK-3β
- GSK-3β ser 9 phosphorylation - indicates GSK-3β inactivation
- PKC Activation - indication of GSK-3β inactivation
- b-catenin - inactivation of the GSK-3β through the activated Wnt Pathway
- Dvl - a protein upstream of GSK-3β in the Wnt pathway, and an indicator of Wnt pathway activation
- Akt - PI-3K signaling cascade protein which phosphorylates GSK-3β at ser9.
- Erk - Map kinase signaling cascade protein that directly phosphorylates GSK-3β at Ser9.
- P38 MAPK - Map kinase signaling cascade protein that directly phosphorylates GSK-3β at Ser389.
- GSK-3β Tyrosine 216 Phosphorylation - Indicates activation of GSK-3β
- Serum Albumin - has been shown to be potentially linked to GVHD onset
- Markers for apoptosis (Fas, Bcl family, Cyctochrome C, caspases)
- Markers for immune activity (Nf-kB, CD25)
- Markers for proliferation (Cyclin D1, PCNA, p27)

In further embodiments of the invention, the protein chip results are further validated by performing a comparison with western blot data to determine the efficacy, the sensitivity, and the accuracy of the chip.

The abovementioned information is used to assess the pathways which are regulating GSK-3β in GVHD and other pathways involved in this disease.

It is a main aspect of the invention that the changes seen in the GSK-3β expression levels and activation state are herein utilized as novel markers for diagnosing the GVHD. By monitoring the changes in the GSK-3β expression levels and changes in the activation state of the GSK-3β protein, the early onset of GVHD can be detected prior to observable clinical symptoms.

In another preferred embodiment of the invention, the use of GSK-3β as a marker allows the diagnosis of GVHD prior to the phase where observable symptoms are demonstrated by the patients and prior to the phase where the host body is attacked by the T-cells.

In further embodiments of the invention, Western blot analysis of mouse splenocyte lysates is performed using at least one of the following proteins as potential markers for GVHD:
- b-catenin - a marker for the inactivation of the GSK-3β through the activated Wnt Pathway.
- Dvl - a protein upstream of GSK-3β in the Wnt pathway, and an indicator of Wnt pathway activation.
- Akt - PI-3K signaling cascade protein which phosphorylates GSK-3β at ser9.
- Erk - Map kinase signaling cascade protein that directly phosphorylates GSK-3β at Ser9.
- P38 MAPK - Map kinase signaling cascade protein that directly phosphorylates GSK-3β at Ser389.
- GSK-3β tyr216 phosphorylation - Indicates the activation of the GSK-3β.

In further embodiments of the invention, Western blot analysis of mouse blood lysates is performed using GSK-3β, p-GSK-3β, PKC, and at least one of the antibodies listed above as potential markers for GVHD.

In a further embodiment of the invention, livers from GVHD model mice are used to perform analysis of GSK-3β and related proteins using western blot and/or mass spectrometry techniques or any other technique used for protein analysis.

It is also within the scope of the present invention that the regulation of GSK-3β expression is tested in a time-course study of an acute GVHD mouse model. Thus by GSK-3β manipulation GVHD onset can be prevented.

In a further embodiment of the invention, the substrates of the GSK-3β and the products associated with said substrates are identified.

In a further embodiment of the invention, the aforementioned substrates and products which are specific for the GVHD disorder are found. Those substrates and products are used for increasing the sensitivity of the diagnostic technology provided by the present invention.

In a further embodiment of the invention the specific pathway that stimulates the specific change in the activation state of the GSK-3β associated with the specific disorder of interest such as GVHD is identified. Such candidates that stimulate the activation state of the GSK-3β may include:
- Dsh - Wnt pathway (upstream of GSK-3β) Associated with inactivation of the GSK-3β through the activated Wnt pathway
- Akt - PI-3K/Akt pathway Associated with Ser 9 inactivation of the Wnt pathway
- cAMP - Associated with Ser 9 inactivation of the GSK-3β

The above described information indicates the pathways that are regulating the activation state of the GSK-3β in the specific disorder of interest such as GVHD. These results are used for fingerprinting a specific disease associated with activation state of the GSK-3β. Such fingerprinting data is used to increase the sensitivity of the diagnostic kit for the disease of interest.

### EXAMPLE 4

### Clinical study using the GVHD diagnostic kit

The following clinical study is conducted in association with a CRA (clinical research associate), including Helsinki approval and negotiations with medical centers:
- Patients for the clinical trial are recruited (including Helsinki approval and negotiations with medical centers).
- Blood samples are collected from the patients who have undergone bone marrow transplants (throughout the year).
- The protein chip is utilized for analysis of the human samples to test its efficacy in predicting acute GVHD onset. This is a retrospective study. The kit analysis results are compared against the diagnostic outcome (according to current medical standards) for each patient.
- If necessary, the kit is calibrated by assaying human samples using western blot analysis.

## Claims

1. A method of detecting Graft versus Host Disease, GVHD, in a mammal including a human comprising the steps of;
measuring the level of GSK-3β in a sample obtained from said mammal, wherein a significant deviation from normal values indicates the presence of GVHD.

2. The method according to claim 1 further comprising one step of measuring the level of GSK-3β by measuring at least one parameter selected from the group consisting of: GSK-3β expression levels, GSK-3β activation levels, GSK-3β inactivation levels, serine 9 phosphorylated GSK-3β expression and p-PKC expression.

3. The method according to claim 1, further comprising a step of measuring the level of GSK-3β expression and/or activation by measuring at least one activation indicator selected from the group consisting of: over-expression of GSK-3β, tyrosine 216 phosphorylation and any combination thereof.

4. The method according to claim 1, further comprising one step of determining the expression and/or inactivation state of GSK-3β by measuring at least one inactivation indicator selected from the group consisting of: (i) serine 9 phosphorylation; (ii) Wnt activation as determined by at least one of: β-catenin levels or by a protein marker specific for inactivation of the GSK-3β through the activated Wnt pathway, or by the site on the GSK-3β that the activated Wnt pathway affects by stimulating inactivation of the GSK-3β; (iii) PKC expression levels (iv) a protein marker specific for inactivation of the GSK-3β through activated PKC, and any combination thereof.

5. The method according to claim 1, comprising an additional step of sampling from either the spleen, the blood, another body fluid or tissue.

6. The method according to claim 1, wherein one of the following folds true: (a) the method further comprising an additional step of comparing the result of said GSK-3β levels with a predetermined normal value obtained from healthy subjects, and (b) a deviation of at least about 25 % from normal inactivation value indicates the presence of GVHD.

7. The method according to claim 1, comprising additional steps of;
a. measuring the level of GSK-3β inactivation by one parameter selected from the group consisting of measuring β-catenin levels in the absence of serine 9 phosphorylation, of measuring the site of stimulation on the GSK-3β once stimulated through the activated Wnt pathway, of measuring a protein marker that is unique to the inactivation of GSK-3β through the activated Wnt pathway and of measuring a protein marker that is unique to the inactivation of GSK-3β through the activated PKC; and
b. comparing the result of said inactivation levels with a predetermined normal inactivation value obtained from healthy subjects,
wherein a deviation of at least about 25 % from normal inactivation values indicates the presence of GVHD.

8. The method according to claim 1, comprising the steps of;
a. measuring GSK-3β inactivation indicators,
b. measuring GSK-3β activation indicators,
c. combining measurements of activation and inactivation indicators,
wherein an activation to inactivation ratio is obtained, characteristic of GVHD.

9. The use of a kit in the diagnosis of Graft versus Host Disease, GVHD, said kit comprising a protein chip comprising an array of bound antibodies recognizing biomarkers characteristic of GVHD, said biomarkers are selected from the group comprising Total GSK-3β, serine 9 phosphorylated GSK-3β, GSK-3β serine 9 phosphorylation PKC activation, GSK-3β tyrosine 216 phosphorylation, for determining the expression levels of different GVHD relevant proteins in a given sample.

10. The use of claim 9, wherein said biomarkers are detectable before appearance of GVHD symptoms in a mammal including a human.

## Patentansprüche

1. Verfahren zum Nachweis von Transplantat-gegen-Wirt-Reaktion, GvHD, bei einem Säuger, einschließlich eines Menschen, umfassend folgende Schritte:
Messen des GSK-3β-Spiegels in einer von besagtem Säuger erhaltenen Probe, wobei eine signifikante Abweichung von Normalwerten das Vorhandensein von GvHD anzeigt.

2. Verfahren nach Anspruch 1, weiter umfassend einen Schritt des Messens des GSK-3β-Spiegels durch Messen mindestens eines Parameters, ausgewählt aus der Gruppe, bestehend aus: GSK-3β-Expressionsspiegeln, GSK-3β-Aktivierungsspiegeln, GSK-3β-Inaktivierungsspiegeln, Expression von Serin-9-phosphoryliertem GSK-3β und p-PKC-Expression.

3. Verfahren nach Anspruch 1, weiter umfassend einen Schritt des Messens des Niveaus von GSK-3β-Expression und/oder -Aktivierung durch Messen mindestens eines Aktivierungsindikators, ausgewählt aus der Gruppe, bestehend aus: Über-Expression von GSK-3β, Tyrosin-216-Phosphorylierung und gleich welcher Kombination davon.

4. Verfahren nach Anspruch 1, weiter umfassend einen Schritt des Bestimmens des Expressions- und/oder Inaktivierungsstatus von GSK-3β durch Messen mindestens eines Inaktivierungsindikators, ausgewählt aus der Gruppe, bestehend aus: (i) Serin-9-Phosphorylierung; (ii) Wnt-Aktivierung, ermittelt durch mindestens eines von: β-Cateninspiegel oder durch einen Proteinmarker, der spezifisch für Inaktivierung des GSK-3β durch den aktivierten Wnt-Signalweg ist, oder durch die Stelle auf dem GSK-3β, die der aktivierte Wnt-Signalweg durch Stimulierung von Inaktivierung des GSK-3β beeinflusst; (iii) PKC-Expressionsspiegel, (iv) einem Proteinmarker, der spezifisch für Inaktivierung des GSK-3β durch aktiviertes PKC ist, und gleich welcher Kombination davon.

5. Verfahren nach Anspruch 1, umfassend einen zusätzlichen Schritt der Probenahme von entweder der Milz, dem Blut, einer anderen Körperflüssigkeit oder Gewebe.

6. Verfahren nach Anspruch 1, wobei eines der folgenden wahr ist:
(a) das Verfahren umfasst weiter einen zusätzlichen Schritt des Vergleichens des Ergebnisses der GSK-3β-Spiegel mit einem vorgegebenen Normalwert, erhalten von gesunden Subjekten, und
(b) eine Abweichung von mindestens ungefähr 25% vom normalen Inaktivierungswert zeigt das Vorhandensein von GvHD an.

7. Verfahren nach Anspruch 1, umfassend folgende zusätzlichen Schritte:
a. Messen des Spiegels von GSK-3β-Inaktivierung durch einen Parameter, ausgewählt aus der Gruppe, bestehend aus dem Messen von β-Cateninspiegeln in Abwesenheit von Serin-9-Phosphorylierung, dem Messen der Stimulierungsstelle auf dem GSK-3β, sobald es durch den aktivierten Wnt-Signalweg stimuliert ist, dem Messen eines Proteinmarkers, der eindeutig für die Inaktivierung von GSK-3β durch den aktivierten Wnt-Signalweg ist, und dem Messen eines Proteinmarkers, der eindeutig für die Inaktivierung von GSK-3β durch das aktivierte PKC ist; und
b. Vergleichen des Ergebnisses der Inaktivierungsspiegel mit einem vorgegebenen normalen Inaktivierungswert, erhalten von gesunden Subjekten,
wobei eine Abweichung von mindestens ungefähr 25% von normalen Inaktivierungswerten das Vorhandensein von GvHD anzeigt.

8. Verfahren nach Anspruch 1, umfassend folgende Schritte:
a. Messen von GSK-3β-Inaktivierungsindikatoren,
b. Messen von GSK-3β-Aktivierungsindikatoren,
c. Kombinieren von Messungen von Aktivierungs- und Inaktivierungsindikatoren,
wobei ein Verhältnis von Aktivierung zu Inaktivierung erhalten wird, das charakteristisch für GvHD ist.

9. Anwendung eines Kits bei der Diagnose von Transplantat-gegen-Wirt-Reaktion, GvHD, wobei das Kit einen Proteinchip umfasst, der eine Anordnung gebundener Antikörper umfasst, die für GvHD charakteristische Biomarker erkennen, wobei die Biomarker aus der Gruppe ausgewählt sind, umfassend Gesamt-GSK-3β, Serin-9-phosphoryliertes GSK-3β, GSK-3β-Serin-9-Phosphorylierung-PKC-Aktivierung, GSK-3β-Tyrosin-216-Phosphorylierung, zur Ermittlung der Expressionsspiegel verschiedener GvHD-relevanter Proteine in einer bestimmten Probe.

10. Anwendung nach Anspruch 9, wobei die Biomarker vor dem Auftreten von GvHD-Symptomen bei einem Säuger, einschließlich eines Menschen, nachweisbar sind.

## Revendications

1. Procédé de détection de la maladie du greffon contre l'hôte, GVHD, chez un mammifère, y compris un être humain, comprenant l'étape consistant à :
mesurer le taux de la GSK-3β dans un échantillon prélevé auprès dudit mammifère, dans lequel une déviation significative par rapport à des valeurs normales indique la présence de GVHD.

2. Procédé selon la revendication 1, comprenant en outre une étape consistant à mesurer le taux de la GSK-3β en mesurant au moins un paramètre choisi parmi le groupe constitué par : les taux d'expression de la GSK-3β ; les taux d'activation de la GSK-3β ; les taux d'inactivation de la GSK-3β ; l'expression de la GSK-3β à sérine 9 phosphorylée ; et l'expression du p-PKC.

3. Procédé selon la revendication 1, comprenant en outre une étape consistant à mesurer le taux d'expression et/ou d'activation de la GSK-3β en mesurant au moins un indicateur d'activation choisi parmi le groupe constitué par : une surexpression de la GSK-3β ; une phosphorylation de la tyrosine 216 ; et l'une quelconque de leurs combinaisons.

4. Procédé selon la revendication 1, comprenant en outre une étape consistant à déterminer l'expression et/ou l'inactivation de la GSK-3β en mesurant au moins un indicateur d'inactivation choisi parmi le groupe constitué par : (i) une phosphorylation de la sérine 9 ; (ii) une activation de la Wnt comme déterminé par au moins un élément choisi parmi : les taux de la β-caténine ou via un marqueur protéinique spécifique pour l'inactivation de la GSK-3β via la voie activée de la Wnt, ou via le site sur la GSK-3β qu'affecte la voie activée de la Wnt en stimulant une inactivation de la GSK-3β ; (iii) les taux d'expression de la PKC ; (iv) un marqueur protéinique spécifique pour l'inactivation de la GSK-3β via la PKC activée ; et l'une quelconque de leurs combinaisons.

5. Procédé selon la revendication 1, comprenant une étape supplémentaire d'échantillonnage à partir soit de la rate, soit du sang, soit d'un autre liquide organique ou d'un tissu.

6. Procédé selon la revendication 1, dans lequel une des affirmations suivantes est vraie : (a) le procédé comprend en outre une étape supplémentaire consistant à comparer les résultats desdits taux de GSK-3β à une valeur normale prédéterminée que l'on obtient auprès de sujets en bonne santé ; et (b) une déviation à concurrence d'au moins environ 25 % par rapport à une valeur d'inactivation normale indique la présence de GVHD.

7. Procédé selon la revendication 1, comprenant des étapes supplémentaires consistant à :
a. mesurer le taux d'inactivation de la GSK-3β via un paramètre choisi parmi le groupe constitué par : la mesure des taux de β-caténine en l'absence d'une phosphorylation de la sérine 9 ; la mesure du site de stimulation sur la GSK-3β, une fois qu'il est stimulé via la voie activée de la Wnt ; la mesure d'un marqueur protéinique qui est unique pour l'inactivation de la GSK-3β via la voie activée de la Wnt ; et la mesure d'un marqueur protéinique qui est unique pour l'inactivation de la GSK-3β via la PKC activée ; et
b. comparer les résultats desdits taux d'inactivation à une valeur d'inactivation normale prédéterminée que l'on obtient auprès de sujets en bonne santé ;
dans lequel une déviation à concurrence d'au moins environ 25 % par rapport à des valeurs d'inactivation normales indique la présence de GVHD.

8. Procédé selon la revendication 1, comprenant des étapes consistant à :
a. mesurer des indicateurs d'une inactivation de la GSK-3β ;
b. mesurer des indicateurs d'une activation de la GSK-3β ;
c. combiner des mesures d'indicateurs d'activation et d'inactivation ;
dans lequel on obtient un rapport activation/inactivation caractéristique de la GVHD.

9. Utilisation d'un nécessaire dans le diagnostic de la maladie du greffon contre l'hôte, GVHD, ledit nécessaire comprenant une puce à protéines comprenant un jeu ordonné d'anticorps liés reconnaissant des biomarqueurs caractéristiques de la GVHD, lesdits biomarqueurs étant choisis parmi le groupe comprenant : la GSK-3β totale ; la GSK-3β à sérine 9 phosphorylée ; une activation de la PKC/phosphorylation de la sérine 9 de la GSK-3β ; une phosphorylation de la tyrosine 216 de la GSK-3β ; pour la détermination des taux d'expression de différentes protéines GVHD pertinentes dans un échantillon donné.

10. Utilisation selon la revendication 9, dans laquelle lesdits biomarqueurs peuvent être détectés avant l'apparition des symptômes de la GVHD chez un mammifère, y compris un être humain.
